(19) Europäisches Patentamt
European Patent Office
Office européen des brevets

(11) Numéro de publication: **0 015 220**
**B1**

(12) **FASCICULE DE BREVET EUROPEEN**

(45) Date de publication du fascicule du brevet: **12.05.82**

(21) Numéro de dépôt: **80420019.4**

(22) Date de dépôt: **14.02.80**

(51) Int. Cl.³: **C 07 C 85/11**, C 07 C 85/24, C 07 C 87/60, C 07 C 93/14

(54) **Procédé de préparation d'anilines métachlorées.**

(30) Priorité: **15.02.79 FR 7904482**

(43) Date de publication de la demande:
**03.09.80 Bulletin 80/18**

(45) Mention de la délivrance du brevet:
**12.05.82 Bulletin 82/19**

(84) Etats contractants désignés:
**AT BE CH DE FR GB IT LU NL SE**

(56) Documents cités:
**FR - A - 2 162 782**
**FR - A - 2 298 531**

**CHEMICAL ABSTRACTS, vol. 69, n° 7, 12 août 1968 réf. 26891r, page 2494 Columbus, Ohio, USA**

**P. V. MOKROUSOV: "Effect of copper on the catalytic properties of a palladium catalyst".**

(73) Titulaire: **RHONE-POULENC AGROCHIMIE**
**14-20, rue Pierre Baizet**
**F-69009 Lyon (FR)**

(72) Inventeur: **Cordier, Georges**
**50 Chemin des Hermières**
**F-69340 - Francheville (FR)**

(74) Mandataire: **Chretien, François et al,**
**RHONE-POULENC AGROCHIMIE BP 9163**
**F-69263 Lyon Cedex 1 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation d'anilines métachlorées

La présente invention se rapporte à un procédé de préparation d'anilines substituées par le chlore en position méta par action de l'hydrogène sur des composés aromatiques azotés plus fortement halogénés.

La préparation de chloranilines substituées en position méta par réaction de polychloranilines avec de l'hydrogène a été décrite dans le brevet français 2 298 531. Le procédé y décrit nécessite toutefois l'utilisation de pressions élevées et de quantités très importantes d'acide chlorhydrique, ce qui pose de graves problèmes de corrosion.

Un but de l'invention est de fournir un procédé permettant de préparer dés anilines substituées en méta par le chlore avec de bons rendements à partir de composés aromatiques azotés plus fortement halogénés.

Un autre but de l'invention est de fournir un procédé de préparation d'anilines substituées en méta par le chlore, ce procédé pouvant mettre en oeuvre comme réactif de départ aussi bien des composés chlorés nitrés (nitrobenzènes substitués et homologues) que aminés (polychloranilines et homologues).

Un autre but de l'invention est de fournir un procédé de préparation d'anilines métasubstitutées par le chlore mettant en oeuvre des pressions modérées.

Un autre but de l'invention est de fournir un procédé de préparation d'anilines métasubstituées par le chlore mettant en oeuvre des températures de réaction modérées.

Un autre but, encore, de l'invention est de fournir un procédé de préparation d'anilines métasubstituées par le chlore mettant en oeuvre des conditions modérément corrosives.

D'autres buts et avantages de l'invention apparaitront au cours de la description qui va suivre.

Il a maintenant été trouvé que l'on pouvait atteindre ces buts grâce à un procédé de préparation d'anilines substituées, en position méta par du chlore, qui consiste à effectuer une hydrogénation catalyque en phase liquide, en milieu acide, à chaud, sous pression, de dérivés benzéniques azotés et chlorés, en présence de métaux nobles du groupe VIII de la classification periodique, caractérisé en ce que les dérivés benzéniques ont la formule:

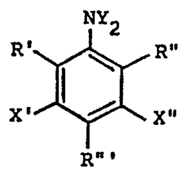

(I)

dans laquelle:

— Y représente l'atome d'hydrogène ou l'atome d'oxygène

— X′ et X″, identiques ou différents entre eux, représentent chacun un atome de chlore ou un radical alkyle ou aryle, ou aralkyle ou alkoxyle ou aralkoxyle, éventuellement substitué, l'un des X′ et X″ pouvant, de plus, être un atome d'hydrogène. Lorsqu'on ,prépare des monochloranilines (métachloranilines), un seul des substituants X′ ou X″ représente l'atome de chlore; lorsqu'on prépare des dichloranilines (dichlorées en méta), les deux symboles X′ et X″ représentent l'atome de chlore.

— R′, R″ et R‴, identiques ou différents entre eux, représentent chacun un atome de chlore ou un radical alkyle ou aralkyle ou alkoxyle ou aryloxyle éventuellement substitué, l'un au moins de ces trois symboles représentant l'atome de chlore, au plus deux des R′, R″ ou R‴ pouvant de plus être l'hydrogène,

et en ce que la réaction est effectuée en présence de cations métalliques lourds, les cations métalliques lourds utilisés appartiennent à l'une des colonnes 1b à 5a de la classification périodique des éléments (numérotation des colonnes conforme à l'édition de 1964 de "Handbook of Chemistry and Physics" page B—2).

La réaction s'effectue, comme il a été dit, en phase liquide; en pratique on opère avantageusement en présence d'un solvant minérale ou organique, liquide et inerte dans les conditions opératoires. Par solvant inerte on entend un solvant ne réagissant pas chimiquement. En fait on préfère utiliser l'eau.

L'acidité du milieu réactionnel est généralement telle que le pH (en cas de milieu aqueux) est avantageusement inférieur à 1,5, de préférence inférieur à 1. La concentration en ions $H^+$ dans le milieu est généralement comprise entre 0,5 et 12 ion-g/l et de préférence entre 1 et 6 ion g. $H^+$/l. Les concentrations les plus élevées en acide peuvent être utilisées mais sans avantage important.

L'acidité du milieu réactionnel peut être obtenue par des acides forts minéraux tels que les acides sulfurique, phosphorique ou halohydriques, ou organiques; mais l'on préfère l'utilisation des acides halohydriques et plus spécialement de l'acide chlorhydrique. De toute manière, étant donné la présence d'ions chlorure issus de la déshalogénation on opère en fait en présence, au moins partiellement, d'acide chlorhydrique.

Le procédé selon l'invention s'effectue en phase liquide (hormis bien sûr, le catalyseur à base de métal noble qui, le plus souvent, constitue une phase solide). La phase liquide peut être homogène et

constituer une solution: c'est là une modalité préférée, spécialement lorsque Y est l'atome d'oxygène dans la formule (I); une telle phase liquide contient donc les réactifs, les produits de réaction et le ou les solvants éventuellement présents. Il est aussi possible d'opérer avec deux phases liquides.

La pression à laquelle s'effectue la réaction est généralement supérieure à 3 bars (pression relative) et de préférence supérieure à 5 bars. Il n'y a pas de limite supérieure critique pour la pression mais pour des raisons d'ordre économique Il est généralement avantageux d'opérer à des pressions inférieures à 100 bars, les pressions inférieures à 30 bars étant préférées.

La température de réaction est généralement comprise entre 90 et 300°C, de préférence entre 110 et 200°C. Une température élevée peut, dans le cas où on utilise des acides relativement volatils, impliquer l'existence d'une pression partielle relativement importante pour les composés de la phase vapeur autres que l'hydrogène (par phase vapeur on entend évidemment la phase vapeur surmontant le milieu liquide réactionnel). On choisit généralement les conditions opératoires de manière que la pression partielle d'hydrogène soit comprise entre 10 et 80% de la pression totale (pression relative) et de préférence entre 30 et 60%.

Les métaux nobles constituant la base des catalyseurs utilisés dans l'invention sont principalement des métaux du groupe VIII de la classification périodique tels que le ruthénium, le rhodium, le palladium, l'osmium, l'iridium et le platine; le palladium est le métal préféré. Le métal peut être à l'état métallique ou à l'état de composé chimique; généralement on préfère que le métal soit mis en oeuvre à l'état métallique car, dans les conditions opératoires, les composés ont tendance à être réduit à l'état métallique (degré d'oxydation = zéro). Le catalyseur peut être supporté ou non supporté. Comme support du catalyseur on peut utiliser tout support connu en soi pour supporter des catalyseurs, pourvu que ce support soit résistant à l'eau et aux acides; comme support convenant plus particulièrement, on peut citer le noir de carbone, la silice, le sulfate de baryum; le charbon actif est un support préféré. Le catalyseur ainsi que son support sont avantageusement sous forme finement divisée; des surfaces spécifiques supérieures à 100 $m^2/g$ conviennent généralement bien.

La quantité de catalyseur mise en oeuvre est telle que la proportion pondérale de métal noble du catalyseur par rapport au composé de formule (I) à traiter est généralement comprise entre 0,05 et 10%, de préférence entre 0,5 et 5%.

Les cations métalliques lourds utilisés dans l'invention ont, en pratique, un rôle de catalyseur favorisant la formation d'amines métachlorées. On peut dire que le procédé de l'invention fonctionne avec un double système catalytique; le premier système catalytique a déjà été décrit et est à base de métal noble (groupe VIII de la classification); le second système catalytique est à base de métaux lourds des groupes 1b à 5a de la classification périodique et a un rôle favorisant la formation d'amines métachlorées; les cations préférés sont ceux à base de bismuth, de plomb, d'étain, de thallium, de mercure et d'argent; comme cations de ce type on peut citer plus particulièrement $Bi^{+++}$, $Pb^{++}$, $Sn^{++}$, $Sn^{++++}$, $Tl^{+}$, $Tl^{+++}$, $Hg^{+}$, $Hg^{++}$, $Ag^{+}$. Ces cations métalliques lourds peuvent être introduits sous les formes les plus diverses, spécialement des sels (par exemple halogénures, sulfates, phosphates) mais aussi des composés à caractère moins salin ou non salin tels que les sulfures et les oxydes. Mais dans les conditions opératoires à caractère très réducteur, ces diverses formes se transforment le plus souvent in situ en cation au degré d'oxydation le plus base. Les quantités de cation métallique lourd dans le milieu réactionnel sont le plus souvent comprises entre 0,00001 et 0,1 atomes grammes/l et de préférence entre 0,001 et 0,05 atomes grammes/l.

L'utilisation de faibles quantités de ces cations métalliques lourds fait qu'il est généralement superflu de les récupérer en fin de réaction, hormis le cas de risques de pollution.

Comme composés de formule (I) susceptibles d'être traités par le procédé de l'invention on peut citer de préférence:

le dichloro-2,3-nitrobenzène et la dichloro-2,3 aniline;
le dichloro-2,5-nitrobenzène et la dichloro-2,5 aniline;
le dichloro-3,4 nitrobenzène et la dichloro-3,4 aniline;
le trichloro-2,3,4 nitrobenzène et la trichloro-2,3,4 aniline;
le trichloro-2,3,5 nitrobenzène et la trichloro-2,3,5 aniline;
le trichloro-2,3,6 nitrobenzène et la trichloro-2,3,6 aniline;
le trichloro-2,4,5 nitrobenzène et la trichloro-2,4,5 aniline;
le trichloro-3,4,5 nitrobenzène et la trichloro-3,4,5 aniline;
le tétrachloro-2,3,4,6 nitrobenzène et la tétrachloro-2,3,4,6 aniline;
le tétrachloro-2,3,4,5 nitrobenzène et la tétrachloro-2,3,4,5 aniline;
le tétrachloro-2,3,5,6 nitrobenzène et la tétrachloro-2,3,5,6 aniline;
le pentachloro-nitrobenzène et la pentachloroaniline;

mais aussi:
le trichloro-4,5,6 méthyl-2 nitrobenzène et la trichloro-4,5,6 méthyl-2 aniline;
le dichloro-2,5 méthyl-4 nitrobenzène et la dichloro-2,5 méthyl-4 aniline;
le tétrachloro-2,3,5,6 méthyl-4 nitrobenzène et la tétrachloro-2,3,5,6 méthyl-4 aniline;
le dichloro-2,5 diméthyl-3,4 nitrobenzène et la dichloro-2,5 diméthyl-3,4 aniline;

le dichloro-2,5 éthyl-4 nitrobenzène et la dichloro-2,5 éthyl-4 aniline;
le dichloro-2,5 propyl-4 nitrobenzène et la dichloro-2,5 propyl-4 aniline;
le trichloro-3,4,6 benzyl-2 nitrobenzène et la trichloro 3,4,6 benzyl-2 aniline;
le dinitro-2,2' hexachloro-3,5,6,3',5',6' diphénylméthane et le diamino-2,2' hexachloro-3,5,6,3',5',6' diphénylméthane;
le nitro-2 trichloro-3,4,5 diphényle et l'amino-2 trichloro-3,4,5 diphényle;
le dinitro-4,4' octachloro diphényle et le diamino-4,4' octachloro diphényle;
le dichloro-4,5 méthoxy-2 nitrobenzène et la dichloro-4,5 méthoxy-2 aniline;
le dichloro-3,4 méthoxy-2 nitrobenzène et la dichloro-3,4 méthoxy-2 aniline;
le dichloro-3,6 méthoxy-2 nitrobenzène et la dichloro-3,6 méthoxy-2 aniline;
le dichloro-5,6 méthoxy-2 nitrobenzène et la dichloro-5,6 méthoxy-2 aniline;
le trichloro-3,4,6 méthoxy-2 nitrobenzène et la trichloro-3,4,6 méthoxy-2 aniline;
le trichloro-3,4,5 méthoxy-2 nitrobenzène et la trichloro-3,4,5 méthoxy-2 aniline;
le tétrachloro-3,4,5,6 méthoxy-2 nitrobenzène et la tétrachloro-3,4,5,6 méthoxy-2 aniline;
le dichloro-4,5 méthoxy-3 nitrobenzène et la dichloro-4,5 méthoxy-3 aniline;
le dichloro-5,6 méthoxy-3 nitrobenzène et la dichloro-5,6 méthoxy-3 aniline;
le dichloro-2,5 méthoxy-3 nitrobenzène et la dichloro-2,5 méthoxy-3 aniline;
le trichloro-4,5,6 méthoxy-3 nitrobenzène et la trichloro-4,5,6 méthoxy-3 aniline;
le tétrachloro-2,4,5,6 méthoxy-3 nitrobenzène et la tétrachloro-2,4,5,6 méthoxy-3 aniline;
le dichloro-2,3 méthoxy-4 nitrobenzène et la dichloro-2,3 méthoxy-4 aniline;
le dichloro-2,5 méthoxy-4 nitrobenzène et la dichloro-2,5 méthoxy-4 aniline;
le tricyhloro-2,3,6 méthoxy-4 nitrobenzène et la trichloro-2,3,6 méthoxy-4 aniline;
le trichloro-2,3,5 méthoxy-4 nitrobenzène et la trichloro-2,3,5 méthoxy-4 aniline;
le tétrachloro-2,3,5,6 méthoxy-4 nitrobenzène et la tétrachloro-2,3,5,6 méthoxy-4 aniline;
le dichloro-4,5 phénoxy-2 nitrobenzène et la dichloro-4,5 phénoxy-2 aniline;
le tétrachloro-3,4,5,6 phénoxy-2 nitrobenzène et la tétrachloro-3,4,5,6 phénoxy-2 aniline;
le tétrachloro-2,4,5,6 phénoxy-3 nitrobenzène et la tétrachloro-2,4,5,6 phénoxy-3 aniline;
le dichloro-2,5 phénoxy-4 nitrobenzène et la dichloro-2,5 phénoxy-4 aniline;
le tétrachloro-2,3,5,6 phénoxy-4 nitrobenzène et la tétrachloro-2,3,5,6 phénoxy-4 aniline.

Parmi les anilines substituées en position méta par l'atome de chlore et susceptibles d'être préparées par le procédé selon l'invention, on peut citer de préférence: la métachloraniline; la dichloro-3,5 aniline mais aussi: la chloro-5 méthyl-2 aniline; la chloro-5 méthyl-3 aniline; la chloro-3 méthyl-4 aniline; la dichloro-3,5 méthyl-4 aniline; la chloro-5 diméthyl-3,4 aniline; la chloro-3 éthyl-4 aniline; la chloro-3 benzyl-2 aniline; le diamino-4,4' tétrachloro-2,6,2',6' diphényle; la chloro-3 méthoxy-2 aniline; la chloro-5 méthoxy-2 aniline; la dichloro-3,5 méthoxy-2 aniline; la chloro-3 méthoxy-4 aniline; la chloro-5 méthoxy-3 aniline; la dichloro-3,5 méthoxy-4 aniline; la chloro-3 phénoxy-2 aniline; la chloro-5 phénoxy-2 aniline; la dichloro-3,5 phénoxy-2 aniline; la dichloro-3,5 phénoxy-4 aniline.

Le procédé selon l'invention peut être effectué en continu ou en discontinu. En fin de réaction le catalyseur peut être séparé, le cas échéant, par filtration ou par des moyens équivalents tels que l'essorage; l'amine métachlorée préparée peut être séparée par tout moyen connu en soi par exemple par extraction à l'aide d'un solvant et/ou par distillation; avant de procéder à cette séparation il convient généralement de faire repasser l'amine (salifiée en milieu acide) sous forme amine (non salifiée) par neutralisation ou alcalinisation à l'aide d'un agent alcalin.

Le procédé selon l'invention est très avantageux en raison de sa bonne sélectivité en amine métachlorée et des conditions relativement douces qu'il permet de réaliser. Les amines métachlorées ainsi produites sont utilisables notamment pour fabriquer des pesticides.

Les exemples donnés à titre non limitatif, illustrent l'invention et montrent comment elle peut être mise en oeuvre.

Exemples 1 à 6

Dans un autoclave de 250 $cm^3$ revêtu intérieurement de tantale, on charge:

— 0,416 g de tétrachloro-2,3,4,5 aniline

— 0,14 g d'un catalyseur constitué de palladium déposé sur du charbon actif (surface spécifique du noir de carbone: 1100 $m^2/g$; teneur pondérale en palladium: 10%)

— 120 $cm^3$ d'une solution aqueuse d'acide chlorhydrique de concentration 4 moles/l

— un sel métallique en quantité telle que la concentration en atome-g/l du métal (à l'état de cation) considéré soit celle donnée dans le tableau I.

L'autoclave est fermé, purgé d'abord à l'argon puis à l'hydrogène. On chauffe alors à 160°C en laissant croître la pression autogène, puis, lorsque cette température est atteinte, on introduit l'hydrogène jusqu'à une pression totale (relative) de 13 bars dont 6 bars de pression partielle d'hydrogène.

On laisse réagir dans ces conditions pendant la durée de réaction indiquée au tableau I. On refroidit; la masse réactionnelle liquide est alcalinisée par une solution aqueuse de soude (NaOH); le catalyseur est séparé par filtration; la dichloro-3,5 aniline est extraite de la phase aqueuse à l'aide de

chlorure de méthylène; la solution dans le chlorure de méthylène ainsi obtenue est séchée sur sulfate de sodium; le solvant est évaporé sous vide. Dans les divers exemples le taux de transformation de la tétrachloraniline a été de 100%. Le rendement en dichloro-3,5 aniline obtenu pour chacun des exemples est indiqué au tableau I

TABLEAU I

| Exemples | Sel métallique | | Durée de la réaction | Rendement en % |
|---|---|---|---|---|
| | Nature | Concentration du cation en at–g / l | | |
| 1 | Tl Cl | 0,005 | 2 h 20 mn | 96,8 |
| 2 | $Pb\ SO_4$ | 0,05 | 2 h 30 mn | 95,7 |
| 3 | $Sn\ Cl_2$ | 0,005 | 3 h | 92,7 |
| 4 | $Bi\ Cl_3$ | 0,005 | 3 h 30 mn | 98,4 |
| 5 | $Ag_2SO_4$ | 0,005 | 3 h 55 mn | 98,1 |
| 6 | $HgCl_2$ | 0,001 | 7 h 30 mn | 97,8 |

**Revendications**

1. Procédé de préparation d'anilines substituées en position méta par du chlore, par hydrogénation catalytique en phase liquide, en milieu acide, à chaud et sous pression, de dérivés benzéniques azotés et chlorés, en présence de métaux nobles du groupe VIII de la classification périodique, caractérisé en ce que les dérivés benzéniques ont la formule:

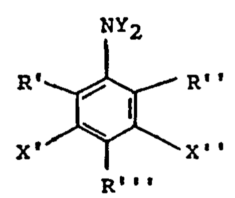

(I)

dans laquelle

— Y représente l'atome d'hydrogène ou l'atome d'oxygène,

— X' et X'', identiques ou différents entre eux, représentent chacun un atome de chlore ou un radical alkyle ou aryle ou aralkyle ou alkoxyle ou aralkoxyle éventuellement substitué, l'un des X' et X'' pouvant de plus être l'hydrogène,

— R', R'' et R''', identiques ou différents entre eux, représentent chacun un atome de chlore ou un radical alkyle ou aralkyle ou alkoxyle ou aryloxyle éventuellement substitué, l'un au moins de ces trois symboles représentant l'atome de chlore, au plus deux des R', R'' ou R''' pouvant de plus être l'hydrogène,

et en ce que la réaction est effectuée en présence de cations métalliques lourds appartenant à l'une des colonnes 1b à 5a de la classification périodique.

2. Procédé selon la revendication 1 caractérisé en ce que le cation métallique est dérivé du bismuth ou du plomb ou de l'étain ou du thallium ou du mercure ou de l'argent.

3. Procédé selon la revendication 2 caractérisé en ce que le cation métallique est l'un des cations $Bi^{+++}$, $Pb^{++}$, $Sn^{++}$, $Sn^{++++}$, $Tl^{+}$, $Tl^{+++}$, $Hg^{+}$, $Hg^{++}$, $Ag^{+}$.

4. Procédé selon l'une des revendications 1 à 3 caractérisé en ce que la concentration en cation métallique lourd est comprise entre 0,00001 et 0,1 atome/l, et de préférence entre 0,001 et 0,05 atome/l.

5. Procédé selon l'une des revendications 1 à 4 caractérisé ence que R', R″, R‴, X', X″ identiques ou différents entre eux représentent l'atome d'hydrogène ou l'atome de chlore.

6. Procédé de préparation de dimétachloroanilines, éventuellement substituées, selon l'une des revendications 1 à 5 caractérisé en ce que X' et X″ représentent l'atome de chlore.

7. Procédé de préparation de monométachloroanilines, éventuellement substituées, selon l'une des revendications 1 à 5 caractérisé en ce que un seul des deux radicaux X' et X″ est l'atome de chlore.

8. Procédé de préparation de dichloro-3,5 aniline selon les revendications 1 à 6 caractérisé en ce que

Y est l'atome d'hydrogène ou d'oxygène

X' et X″ sont l'atome de chlore

R', R″, R‴ sont l'atome d'hydrogène ou l'atome de chlore, l'un d'entre eux au moins étant l'atome de chlore.

9. Procédé selon l'une des revendications 1 à 8 caractérisé en ce que le pH est inférieur à 1,5 et/ou que la concentration en ions $H^+$ du milieu réactionnel est comprise entre 0,5 et 12 ion-g/l.

10. Procédé selon la revendication 9 caractérisé en ce que le pH est inférieur à 1 et/ou que la concentration en ions $H^+$ du milieu réactionnel est comprise entre 1 et 6 ion-g/l.

11. Procédé selon l'une des revendications 1 à 10 caractérisé en ce que le milieu réactionnel est un milieu aqueux.

12. Procédé selon l'une des revendications 1 à 11 caractérisé en ce que le milieu réactionnel ne comporte qu'une phase liquide hormis le catalyseur à base de métal noble.

13. Procédé selon l'une des revendications 1 à 12 caractérisé en ce que la pression totale est comprise entre 3 et 100 bars.

14. Procédé selon la revendication 13 caractérisé en ce que la pression totale est comprise entre 5 et 30 bars.

15. Procédé selon l'une des revendications 1 à 14 caractérisé en ce que la température est comprise entre 90 et 300°C, de préférence entre 110 et 200°C.

16. Procédé selon l'une des revendications 1 à 15 caractérisé en ce que la pression partielle d'hydrogène est comprise entre 10 et 80% de la pression totale.

17. Procédé selon la revendication 16 caractérisé en ce que la pression partielle d'hydrogène est comprise entre 30 et 60% de la pression totale.

18. Procédé selon la revendication 1 caractérisé en ce que la catalyseur est le palladium.

19. Procédé selon l'une des revendications 1 à 18 caractérisé en ce que la proportion pondérale de métal noble par rapport au composé de formule (I) est comprise entre 0,05 et 10%, de préférence entre 0,5 et 5%.

**Patentansprüche**

1. Verfahren zur Herstellung m-chlorsubstituierter Aniline durch katalytische Hydrierung von stickstoffhaltigen und chlorierten Benzolderivaten in flüssiger Phase in einem sauren Medium in der Wärme und unter Druck in Gegenwart von Edelmetallen der Gruppe VIII des Periodensystems, dadurch gekennzeichnet, daß (a) als Benzolderivate Verbindungen der Formel I

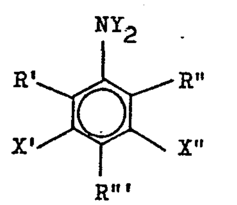

(I)

verwendet werden, in der bedeuten:

Y Wasserstoff oder Sauerstoff,

X' und X″ zugleich oder unabhängig Chlor, Alkyl, Aryl, Aralkyl, Alkoxy oder Aralkoxy, die ggf substituiert sind, wobei einer der beiden Substituenten X' und X″ auch Wasserstoff bedeuten kann, und

R', R″ und R‴ zugleich oder unabhängig Chlor, Alkyl, Aralkyl, Alkoxy oder Aryloxy, die ggf substituiert sind, wobei mindestens einer der Substituenten R', R″ und R‴ Chlor ist und ferner höchstens zwei dieser Substituenten Wasserstoff bedeuten können, und

(b) die Reaktion in Gegenwart von Kationen von Schwermetallen aus einer der Gruppen 1b bis 5a des Periodensystems durchgeführt wird.

2. Verfahren nach Anspruch 1, dadurch gekennzeichnet, daß als Metallkation Wismut-, Blei-, Zinn-, Thallium-, Quecksilber- oder Silberionen verwendet werden.

3. Verfahren nach Anspruch 2, dadurch gekennzeichnet, daß als Metallkation $Bi^{3+}$, $Pb^{2+}$, $Sn^{2+}$, $Sn^{4+}$, $Tl^{+}$, $Tl^{3+}$, $Hg^{+}$, $Hg^{2+}$ oder $Ag^{+}$ verwendet wird.

4. Verfahren nach einem der Ansprüche 1 bis 3, dadurch gekennzeichnet, daß die Schwermetallkationen in einer Konzentration von $10^{-5}$ bis $10^{-1}$ Gramm-atom/l, vorzugsweise von $10^{-3}$ bis $5 \times 10^{-2}$

Gramm-atoms eingesetzt werden.

5. Verfahren nach einem der Ansprüche 1 bis 4, dadurch gekennzeichnet, daß ein Benzolderivat der Formel I verwendet wird, in der R′, R″, R‴, X′ und X″ zugleich oder unabhängig Wasserstoff oder Chlor bedeuten.

6. Verfahren nach einem der Ansprüche 1 bis 5 zur Herstellung ggf substituierter Di-m-chloraniline, dadurch gekennzeichnet, daß eine Verbindung der Formel I eingesetzt wird, in der X′ und X″ Chlor bedeuten.

7. Verfahren nach einem der Ansprüche 1 bis 5 zur Herstellung ggf substituierter Mono-m-chloraniline, dadurch gekennzeichnet, daß eine Verbindung der Formel I verwendet wird, in der nur einer der Substituenten X′ und X″ Chlor bedeutet.

8. Verfahren nach einem der Ansprüche 1 bis 6 zur Herstellung von 3.5-Dichloranilin, dadurch gekennzeichnet, daß eine Verbindung der Formel I verwendet wird, in der bedeuten:

Y Wasserstoff oder Sauerstoff,

X′ und X″ Chlor und

R′, R″ und R‴ Wasserstoff oder Chlor, wobei mindestens einer der Substituenten R′, R″ und R‴ Chlor bedeutet.

9. Verfahren nach einem der Ansprüche 1 bis 8, dadurch gekennzeichnet, daß bei einem pH-Wert des Reaktionsmediums unter 1,5 und/oder einer $H^+$-Ionenkonzentration des Reaktionsgemischs zwischen 0,5 und 12 val/l gearbeitet wird.

10. Verfahren nach Anspruch 9, dadurch gekennzeichnet, daß bei einem pH-Wert des Reaktionsmediums unter 1 und/oder einer $H^+$-Ionenkonzentration des Reaktionsmediums von 1 bis 6 val/l gearbeitet wird.

11. Verfahren nach einem der Ansprüche 1 bis 10, dadurch gekennzeichnet, daß als Reaktionsmedium ein wäßriges Medium verwendet wird.

12. Verfahren nach einem der Ansprüche 1 bis 11, dadurch gekennzeichnet, daß das Reaktionsmedium, abgesehen vom Edelmetallkatalysator, aus lediglich einer flüssigen Phase besteht.

13. Verfahren nach einem der Ansprüche 1 bis 12, dadurch gekennzeichnet, daß ein Gesamtdruck von 3 bis 100 bar angewandt wird.

14. Verfahren nach Anspruch 13, dadurch gekennzeichnet, daß ein Gesamtdruck von 5 bis 30 bar angewandt wird.

15. Verfahren nach einem der Ansprüche 1 bis 14, dadurch gekennzeichnet, daß bei einer Temperatur von 90 bis 300°C vorzugsweise von 110°C bis 200°C verfahren wird.

16. Verfahren nach einem der Ansprüche 1 bis 15, dadurch gekennzeichnet, daß bei einem Wasserstoffpartialdruck von 10 bis 80% des Gesamtdrucks gearbeitet wird.

17. Verfahren nach Anspruch 16, dadurch gekennzeichnet, daß bei einem Wasserstoffpartialdruck von 30 bis 60% des Gesamtdrucks gearbeitet wird.

18. Verfahren nach dem Anspruch 1, dadurch gekennzeichnet, daß als Katalysator Palladium verwendet wird.

19. Verfahren nach einem der Ansprüche 1 bis 18, dadurch gekennzeichnet, daß das Edelmetall des Katalysators in einer Menge von 0,05 bis 10 Gew.-%, vorzugsweise von 0,5 bis 5 Gew.-%, bezogen auf das Gewicht der Verbindung der Formel I, eingesetzt wird.

**Claims**

1. A process for the preparation of anilines substituted in the meta-position by chlorine, by the catalytic hydrogenation of chlorine-substituted nitrogen-containing benzene derivatives, in the liquid phase, in an acid medium, under the action of heat, under pressure and in the presence of noble metals from group VIII of the periodic classification, characterised in that the benzene derivatives have the formula:

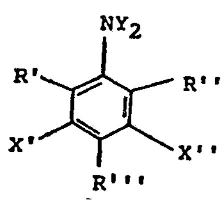

(I)

in which

Y represents the hydrogen atom or the oxygen atom,

X′ and X″, which are identical or different from one another, each represent a chlorine atom or an optionally substituted alkyl, aryl, aralkyl, alkoxy or aralkoxy radical, it being furthermore possible for one of the symbols X′ and X″ to be hydrogen, and

R′, R″ and R‴, which are identical or different from one another, each represent a chlorine atom or an optionally substituted alkyl, aralkyl, alkoxy or aryloxy radical, at least one of these three symbols representing the chlorine atom and it being furthermore possible for at most two of the symbols R′, R″

or R''' to be hydrogen, and that the reaction is carried out in the presence of heavy metal cations belonging to one of columns 1b to 5a of the periodic classification.

2. A process according to claim 1, characterised in that the metal cation is derived from bismuth, lead, tin, thallium, mercury or silver.

3. A process according to claim 2, characterised in that the metal cation is one of the cations $Bi^{+++}$, $Pb^{++}$, $Sn^{++}$, $Sn^{++++}$, $Tl^{+}$, $Tl^{+++}$, $Hg^{+}$, $Hg^{++}$ and $Ag^{+}$.

4. A process according to one of claims 1 to 3, characterised in that the concentration of heavy metal cation is between 0.00001 and 0.1 g.atom/litre and preferably between 0.001 and 0.05 g.atom/litre.

5. A process according to one of claims 1 to 4, characterised in that R', R'', R''', X' and X'', which are identical or different from one another, represent the hydrogen atom or the chlorine atom.

6. A process for the preparation of optionally substituted meta-dichloroanilines, according to one of claims 1 to 5, characterised in that X' and X'' represent the chlorine atom.

7. A process for the preparation of optionally substituted meta-monochloroanilines, according to one of claims 1 to 5, characterised in that only one of the two radicals X' and X'' is the chlorine atom.

8. A process for the preparation of 3,5-dichloroaniline, according to claims 1 to 6, characterised in that

Y is the hydrogen or oxygen atom,

X' and X'' are the chlorine atom and

R', R'' and R''' are the hydrogen atom or the chlorine atom, at least one of them being the chlorine atom.

9. A process according to one of claims 1 to 8, characterised in that the pH is less than 1.5 and/or the concentration of $H^+$ ions in the reaction medium is between 0.5 and 12 g.ions/litre.

10. A process according to claim 9, characterised in that the pH is less than 1 and/or the concentration of $H^+$ ions in the reaction medium is between 1 and 6 g.atoms/litre.

11. A process according to one of claims 1 to 10, characterised in that the reaction medium is an aqueous medium.

12. A process according to one of claims 1 to 11, characterised in that the reaction medium consists only of a liquid phase, except for the catalyst based on a noble metal.

13. A process according to one of claims 1 to 12, characterised in that the total pressure is between 3 and 100 bars.

14. A process according to claim 13, characterised in that the total pressure is between 5 and 30 bars.

15. A process according to one of claims 1 to 14, characterised in that the temperature is between 90 and 300°C and preferably between 110 and 200°C.

16. A process according to one of claims 1 to 15, characterised in that the hydrogen partial pressure is between 10 and 80% of the total pressure.

17. A process according to claim 16, characterised in that the hydrogen partial pressure is between 30 and 60% of the total pressure.

18. A process according to claim 1, characterised in that the catalyst is palladium.

19. A process according to one of claims 1 to 18, characterised in that the proportion by weight of noble metal, relative to the compound of the formula (I), is between 0.05 and 10% and preferably between 0.5 and 5%.